# EUROPÄISCHE PATENTSCHRIFT

(11) **EP 0 801 637 B1**
(45) Veröffentlichungstag und Bekanntmachung des Hinweises auf die Patenterteilung: **06.05.1999**
(21) Anmeldenummer: 96900564.4
(22) Anmeldetag: 04.01.1996
(51) Int. Cl.: C07C 29/88, C07C 31/20

(54) **VERFAHREN ZUR VERBESSERUNG DER HEXANDIOL-1,6-REINHEIT**
PROCESS FOR IMPROVING THE PURITY OF HEXANE DIOL-1,6
PROCEDE D'AMELIORATION DE LA PURETE D'HEXANEDIOL-1,6

(30) Priorität: 05.01.1995 DE 19500236
(43) Veröffentlichungstag der Anmeldung: 22.10.1997
(73) Patentinhaber: BASF AKTIENGESELLSCHAFT, 67056 Ludwigshafen (DE)
(72) Erfinder: DOSTALEK, Roman, D-67354 Römerberg (DE); FISCHER, Rolf, D-69121 Heidelberg (DE); HARDER, Wolfgang, D-69469 Weinheim (DE); PAUL, Axel, D-68623 Lampertheim (DE); PINKOS, Rolf, D-67098 Bad Dürkheim (DE)
(74) Vertreter: Isenbruck, Günter, Dr.
(86) Internationale Anmeldenummer: EP9600023
(87) Internationale Veröffentlichungsnummer: WO9620909

(56) Entgegenhaltungen:
- DE-A- 2 060 548
- US-A- 3 524 892
- US-A- 3 933 930
- PATENT ABSTRACTS OF JAPAN vol. 015 no. 163 (C-0826) ,24.April 1991 & JP,A,03 034946 (UBE IND LTD) 14.Februar 1991,

## Beschreibung

Die vorliegende Erfindung betrifft ein Verfahren zur Herstellung von Hexandiol-1,6 von hoher Reinheit. Insbesondere betrifft die vorliegende Erfindung ein Verfahren, das die Abtrennung von cyclischen Hexandiolen wie 1,4-Dihydroxycyclohexan sowie cyclischen Hydroxyketonen wie 4-Hydroxycyclohexanon aus Lösungen, die Hexandiol-1,6 oder Hexandiol-1,6-Vorläufer wie Adipinsäure, 6-Hydroxycapronsäure und/oder andere Abstreifsäuren (unter Abstreifsäuren seien ganz allgemein Gemische aus Mono- und Dicarbonsäuren verstanden) enthalten, ermöglicht.

Es ist bekannt, daß Hexandiol-1,6, ein vielfach als Monomerbaustein auf dem Polyester-Gebiet verwendetes Diol, technisch durch Hydrierung von Adipinsäure und/oder 6-Hydroxycapronsäure und anschließende Destillation des Hydrieraustrags hergestellt werden kann [Ullmanns Enzyklopädie der technischen Chemie, 4. Auflage, Bd. 7, S. 228 f. (1974)].

Wäßrige Lösungen, bestehend aus Adipinsäure, 6-Hydroxycapronsäure und/oder zahlreichen anderen Mono- und Dicarbonsäuren - sogenannten Abstreifsäuren - entstehen beispielsweise als Koppelprodukt bei der Oxidation von Cyclohexan zu Cyclohexanol/Cyclohexanon-Gemischen und der anschließenden Aufarbeitung des Oxidats. Derartige Lösungen enthalten zwischen 10 und 50 Gew.-% Adipinsäure und 6-Hydroxycapronsäure und etwa 0,1 - 3 Gew.-% Cyclohexandiole und Hydroxycyclohexanone. Diese cyclischen Diole sind gegenüber der Hydrierung relativ stabil und bleiben daher auch nach der Hydrierung der oben erwähnten Säuren zu Hexandiol-1,6 weitgehend erhalten, während die Hydroxycyclohexanone zu Cyclohexandiolen hydriert werden. Auch destillativ lassen sich diese Cyclohexandiole nur sehr schwer von Hexandiol-1,6 abtrennen.

Eine Möglichkeit, diese cyclischen Diole aus den oben genannten, z. B. Adipinsäure enthaltenden Lösungen zu entfernen, wird in dem US-Patent 3,933,930 beschrieben. Danach wird im Anschluß an die Oxidation von Cyclohexan ein katalytischer Schritt der Vorhydrierung eingeschoben, so daß 1,4-Dihydroxycyclohexan (entstanden bei der Oxidation von Cyclohexan) zu Cyclohexanol, Cyclohexan und/oder Cyclohexen umgewandelt wird, ohne daß eine wesentliche Hydrierung der z. B. Adipinsäure und 6-Hydroxyhexansäure erfolgt. Dieser Schritt der Vorhydrierung erfordert jedoch die Verwendung eines Hydrierkatalysators, der von dem Hydrierkatalysator für die eigentliche Reduktion, nämlich für die Reduktion der Adipinsäure und der 6-Hydroxyhexansäure zu Hexandiol-1,6, verschieden ist. Die Vorhydrierung erfolgt in Gegenwart von molekularem Wasserstoff unter hohen Drücken (300 atm), was die Gesamtreaktion zusätzlich aufwendig gestaltet.

Die DE-A-20 60 548 beschreibt ein Verfahren zur Reinigung von Hexandiol-1,6, das geringe Mengen anderer Diole enthält, durch Kristallisation. Die so erzielte Hexandiol-Reinheit wird dort mit 99,8% angegeben. Obwohl dieses Verfahren sehr reines Hexandiol-1,6 liefert, ist es jedoch insofern nachteilig, als es sehr hohe Investitionskosten erfordert.

Es bestand somit die Aufgabe, ein Verfahren zu entwickeln, mit dem sich unerwünschte und schwer abtrennbare cyclische Hexandiole selektiv, ohne großen apparativen Aufwand und relativ billig von Lösungen, die entweder bereits Hexandiol-1,6 oder dessen Vorläuferverbindungen enthalten, abtrennen lassen.

Diese Aufgabe wurde nunmehr erfindungsgemäß durch ein vereinfachtes Verfahren gelöst, wie es im Anspruch 1 definiert ist. Bevorzugt ist dabei ein Verfahren, bei dem
(a) Lösungen von Hexandiol-1,6, die zunächst mit Carbonsäuren versetzt worden sind; oder
(b) Lösungen der Vorläuferverbindungen von Hexandiol-1,6 (wie z.B. der Adipinsäure)
ohne Zugabe eines Hydrierkatalysators und ohne Wasserstoff auf Temperaturen bis 300 °C erhitzt werden. Dadurch können die unerwünschten cyclischen Hexandiole aus den Lösungen (a) und (b) entfernt werden, ohne daß ein Hydrierkatalysator und Wasserstoff wie in dem US-Patent 3,933,930 nötig sind. Demzufolge verringert sich der apparative Aufwand, der betrieben werden muß, und als Folge davon die Kosten für die Herstellung von gereinigtem Hexandiol-1,6.

Weitere bevorzugte Merkmale bzw. Ausführungsformen der Erfindung ergeben sich aus der folgenden Beschreibung und den Beispielen.

Wie bereits erwähnt, fallen bei der katalytischen Oxidation von Cyclohexan zu Cyclohexanol/Cyclohexanon-Gemischen wäßrige Waschlösungen - sogenannte Abstreifsäuren - an, die neben geringen Mengen cyclischer Diole vor allem Adipin- und 6-Hydroxycapronsäure (insgesamt etwa 10 bis 80 Gew.-%) enthalten. Werden diese Waschlösungen nun einer katalytischen Hydrierung und anschließenden Destillation des Hydrierprodukts unterworfen, erhält man Hexandiol-1,6, das cyclische Diole enthält. Dabei handelt es sich im wesentlichen um die cis- und trans-Isomeren von Cyclohexandiol-1,2, Cyclohexandiol-1,3 und Cyclohexandiol-1,4.

Durch Erhitzen der oben genannten Lösungen (a) und (b) werden die störenden Cyclohexandiole bzw. Hydroxycyclohexanone zu einem großen Teil abgebaut, ohne daß das Hexandiol-1,6 oder seine Vorläuferverbindungen zerstört werden. Das Ausmaß, in dem die Cyclohexandiole bzw. Hydroxycyclohexanone zerstört werden, hängt wesentlich von der Temperatur und der Dauer der thermischen Behandlung ab. Die bevorzugte Temperatur liegt bei 130 - 300 °C, wobei der Bereich von 180 - 260 °C besonders bevorzugt ist. Auch die Reaktionszeit, d.h. die Zeitdauer der thermischen Behandlung, beeinflußt das Maß des Abbaus der unerwünschten Cyclohexandiole. Übliche Reaktionszeiten liegen bei wenigen Minuten bis zu mehreren Stunden. Bevorzugte Reaktionszeiten betragen zehn Minuten bis zwanzig Stunden, besonders bevorzugt sind 0,3 - 10 Stunden.

Das Gewichtsverhältnis zwischen zugesetzter Carbonsäure in der Lösung (a) bzw. den Säuren (Vorläuferverbindungen von Hexandiol-1,6) der Lösung (b) auf der einen Seite und den zu beseitigenden Cyclohexandiolen auf der anderen Seite ist nicht weiter kritisch. Das Verhältnis, bezogen auf das jeweilige Gewicht, sollte jedoch zumindest 1 : 1, bevorzugterweise größer gleich 5 : 1, sein, wobei ein Überschuß der Carbonsäuren in (a) bzw. in (b) relativ zu den Cyclohexandiolen bevorzugt ist, da ein solcher Überschuß zu einer Steigerung der Reaktionsgeschwindigkeit führt.

Die Umsetzung der Cyclohexandiole nach dem erfindungsgemäßen Verfahren findet bevorzugterweise in wässrigen Systemen statt. Dementsprechend beeinflußt auch das Gewichtsverhältnis Wasser/Cyclohexandiole den Reaktionsablauf und die Reaktionsgeschwindigkeit. Das Gewichtsverhältnis von Wasser zu Cyclohexandiolen sollte gemäß der vorliegenden Erfindung mindestens 5 : 1 betragen, wobei höhere Gewichtsverhältnisse wie z.B. 15 : 1 bis 30 : 1 oder noch größere Verhältnisse bevorzugt sind. Insbesondere werden Ausgangsgemische erfindungsgemäß erwärmt, die bis zu 5 Gew.-% Cyclohexandiole, bezogen auf das Ausgangsgemisch, also einschließlich Wasser, erhalten.

Im einfachsten Fall wird die Reaktion derart durchgeführt, daß die Lösungen (a) bzw. (b) unter Rühren in einem Druckreaktor auf die oben genannten Temperaturen (130 - 300 °C) erhitzt werden. Die Verweilzeit der jeweiligen Lösung im Druckreaktor bestimmt dabei den Grad der Umsetzung, d.h. der Abreicherung bzw. Entfernung, der Cyclohexandiole. Anstelle dieser diskontinuierlichen Arbeitsweise eignet sich jedoch auch die kontinuierliche Verfahrensweise, bei der die Lösung (a) bzw. (b) in einen beheizten Rührreaktor bzw. in ein Reaktionsrohr gefahren wird und unter Einhaltung der gewünschten Verweilzeit kontinuierlich wieder aus der heißen Reaktionszone entfernt wird.

Geeignete Carbonsäuren, die dem Hexandiol-1,6 zur Herstellung der Lösung (a) zugesetzt bzw. die in dem Gemisch der Vorläuferverbindungen (Lösung (b)) enthalten sein können, sind alle aliphatischen C₁-C₂₀-Monocarbonsäuren, alle C₂-C₁₈-Dicarbonsäuren, aromatische und araliphatische sowie halogenhaltige Carbonsäuren. Dazu zählen bevorzugterweise die Monocarbonsäuren Essig-, Propion-, Butter-, Valerian- und Capronsäure, aber auch die Dicarbonsäuren Oxal-, Malon-, Bernstein-, Glutar-, Adipin- sowie 6-Hydroxycapronsäure. Besonders bevorzugt als Carbonsäuren sind die Adipin- und die 6-Hydroxycapronsäure.

Für die erfindungsgemäße Abtrennung von Cyclohexandiolen aus den oben genannten Lösungen (a) und (b) ist es in aller Regel nicht erforderlich, einen Katalysator zu verwenden. Ein Katalysator kann jedoch dann vorteilhaft sein, wenn besonders kurze Verweilzeiten gewünscht sind. In einem solchen Fall kann die erfindungsgemäße Umsetzung in Gegenwart saurer Katalysatoren wie z.B. Mineralsäuren, Sulfonsäuren, Heteropolysäuren, starksauren Ionenaustauschern, Zeolithen, Alumosilikaten, SiO₂, Al₂O₃, TiO₂ und/oder ZrO₂ erfolgen. Bevorzugt sind saure Oxide wie SiO₂, Al₂O₃, TiO₂ oder Gemische dieser Oxide. Zur Erhöhung der Säurestärke der Oxide können diese mit Sulfat- oder Phosphatgruppen dotiert sein, wobei der Gewichtsanteil 0,5 bis 10% (bezogen auf das Gesamtgewicht) betragen kann.

Wenn die thermisch behandelte Lösung die Lösung (a) war, so ist die Hydrierung der z.B. Adipin- und 6-Hydroxycapronsäure bereits unter den im Stand der Technik bekannten Bedingungen (siehe z.B. DE-AS-12 35 879) durchgeführt worden. Die Umsetzung der störenden Cyclohexandiole im wässrigen Hydrieraustrag erfolgt dann also nach der Reduktion der Säuren zu Hexandiol-1,6 und nach Zugabe der oben genannten Carbonsäuren unter Herstellung der Lösung (a) durch Erhitzen auf hohe Temperaturen (130 - 300 °C), ohne daß ein Hydrierkatalysator und Wasserstoff zugesetzt werden müssen.

Alternativ dazu kann die erfindungsgemäße thermische Behandlung jedoch auch unmittelbar auf die Vorläuferverbindungen von Hexandiol-1,6, d.h. die Lösung (b), angewendet werden. An die thermische Behandlung gemäß der vorliegenden Erfindung schließt sich dann die bekannte Hydrierung mittels eines Hydrierkatalysators (wie z.B. in DE-AS-12 35 879 beschrieben) an. Die auf diese Weise erhaltenen Hydrierausträge können dann destillativ aufgetrennt werden, wobei Hexandiol-1,6 mit deutlich reduziertem Anteil an Cyclohexandiolen erhalten wird.

Die nachfolgend beschriebenen Beispiele erläutern nicht nur das erfindungsgemäße Verfahren, sondern zeigen auch, daß dieses Verfahren außerordentlich geeignet ist, durch Umsetzung von verunreinigenden Cyclohexandiolen eine deutlich reinere Lösung von Hexandiol-1,6 zu erhalten.

### Beispiel 1

150 ml Dicarbonsäurelösung (54 Gewichts-% Wasser, 16,5 Gewichts-% Adipinsäure, 17,8 Gewichts-% 6-Hydroxycapronsäure, 1,8 Gewichts-% Cyclohexandiole und als Rest zu 100 Gew.-% weitere Mono- und Dicarbonsäuren) werden in einem 300 ml Druckreaktor 5 Stunden iang bei 250 °C (Eigendruck 50 bar = 5 x 10⁶ Pa) unter Rühren erhitzt. Der Wassergehalt läßt sich nach der Karl-Fischer-Methode, der Carbonsäure-Gehalt durch HPLC und der Cyclohexandiol-Gehalt nach Trifluoracetylierung der Dicarbonsäure-Lösung mit Trifluoracetanhydrid gaschromatographisch bestimmen.

Nach Versuchsbeendigung wird das Reaktionsgemisch auf Raumtemperatur abgekühlt und wieder analysiert:

| | |
|---|---|
| Wassergehalt | 55 Gewichts-% |
| Adipinsäure-Gehalt | 16,2 Gewichts-% |
| 6-Hydroxycapronsäure-Gehalt | 18,1 Gewichts-% |
| Cyclohexandiol-Gehalt | 0,19 Gewichts-% |
| weitere Mono- und Dicarbonsäuren | Rest auf 100 Gewichts-% |

### Beispiel 2

Analog zu Beispiel 1 erhitzt man eine Dicarbonsäure-Lösung (mit einem Cyclohexandiol-Gehalt von 1,6 Gewichts-%, 17 Gewichts-% Adipinsäure, 17,3 Gewichts-% 6-Hydroxycapronsäure) auf 230 °C. Nach jeweils 5 und 20 Stunden werden Proben gezogen und auf ihren Cyclohexandiol-Gehalt untersucht:

| | |
|---|---|
| Cyclohexandiol-Gehalt nach 5 Std. Versuchszeit | 0,27 Gewichts-% |
| Cyclohexandiol-Gehalt nach 20 Std. Versuchszeit | 0,08 Gewichts-% |

### Beispiel 3

Die Dicarbonsäure-Lösung aus Beispiel 1 wird mit einem kontinuierlichen Zulauf von 40 ml/h (entspricht einer mittleren Verweilzeit von 1,75 Stunden) durch ein 230 °C heißes Reaktionsrohr (Volumen 70 ml) gefahren. Etwa 10 1 der so vorbehandelten Dicarbonsäure-Lösung werden mit einem kontinuierlichen Zulauf von 130 g/h in einen auf 240 °C beheizten 300 ml-Hydrierreaktor gefahren, der 200 ml eines Kobalt-Katalysators enthält. Der Wasserstoffdruck beträgt 250 bar (2,5 x 10⁷ Pa). Die mittlere Verweilzeit der Dicarbonsäure-Lösung im Hydrierreaktor beträgt 2 - 3 Stunden. Eine gaschromatographische Analyse des Hydrieraustrags ergibt:

| | |
|---|---|
| Hexandiol-1,6-Gehalt | 31,3 Gewichts-% |
| Cyclohexandiol-Gehalt | 0,7 Gewichts-% |

### Vergleichsbeispiel

Die Dicarbonsäure-Lösung aus Beispiel 3 wird nicht thermisch vorerhitzt, sondern unter Einhaltung derselben Reaktionsparameter analog zu Beispiel 3 kontinuierlich hydriert. Die GC-Analyse des Hydrieraustrags ergibt folgendes Bild:

| | |
|---|---|
| Hexandiol-1,6-Gehalt | 30,4 Gewichts-% |
| Cyclohexandiol-Gehalt | 1,74 Gewichts-% |

### Beispiel 4

150 g einer Mischung aus 69 Gewichts-% Wasser, 30 Gewichts-% Hexandiol-1,6 und 1 Gewichts-% Cyclohexandiol-1,4 werden mit 1,6 g Hexansäure versetzt und unter Rühren 5 Stunden lang auf 250 °C erhitzt. Die gaschromatographische Analyse des Reaktionsaustrages nach Versuchsbeendigung ergibt einen Cyclohexandiol-1,4-Gehalt von 0,49 Gewichts-%.

### Beispiel 5

Eine Dicarbonsäure-Lösung (55 Gew.-% Wasser, Adipinsäure 17 Gew.-%, 6-Hydroxycapronsäure 15 Gew.-%, 2 Gew.-% Cyclohexandiole und als Rest zu 100 Gew.-% weitere Mono- und Dicarbonsäuren) wird mit einem kontinuierlichen Zulauf von 1200 ml/h durch ein mit 400 ml SiO₂-Strängen (4 mm Durchmesser) gefülltes Reaktionsrohr und anschließend durch einen auf 230 °C beheizten 2,5 1 Hydrierreaktor, der 2,4 ml des in Beispiel 3 erwähnten Co-Katalysator enthält, gefahren. Der Wasserstoffdruck beträgt 260 bar. Die Temperatur im mit SiO₂ gefüllten Reaktionsrohr betrug anfänglich 25 °C. Die Analyse des Hydrieraustrages ergab dabei:

| | |
|---|---|
| Hexandiol-1,6-Gehalt | 23 Gew.-% |
| Cyclohexandiol-Gehalt | 2,3 Gew.-% |

Nach 3 Tagen Versuchszeit wurde die Temperatur im mit SiO₂ gefüllten Reaktionsrohr auf 220 °C erhöht. Die Analyse des Hydrieraustrags ergab dabei:

| | |
|---|---|
| Hexandiol-1,6-Gehalt | 23 Gew.-% |
| Cyclohexandiol-Gehalt | 1,2 Gew.-% |

## Patentansprüche

1. Verfahren zur Abtrennung von Verunreinigungen aus wässrigen Lösungen von Hexandiol-1,6 bzw. Hexandiol-1,6-Vorläuferverbindungen wie Adipin- und 6-Hydroxycapronsäure, wobei das Verfahren die Schritte umfaßt, zu einer Lösung (a) von Hexandiol-1,6 mindestens eine Carbonsäure zu geben und diese Lösung (a) bzw. eine als Vorläuferverbindung(en) des Hexandiol-1,6 Carbonsäure(n) enthaltende Lösung (b) in Abwesenheit von Wasserstoff einer Hitzebehandlung bei Temperaturen oberhalb der Raumtemperatur zu unterwerfen.

2. Verfahren nach Anspruch 1, wobei die zugegebene Carbonsäure(n) eine oder mehrere C₂- bis C₂₀-Monocarbonsäure(n), C₂- bis C₁₈-Dicarbonsäure(n) und/oder ein Gemisch von diesen ist, wobei diese Mono- und Dicarbonsäuren aromatische Reste, cycloaliphatische Reste, heterocyclische Reste und/oder Halogenatome enthalten können.

3. Verfahren nach Anspruch 1 oder 2, wobei die zugegebene Carbonsäure die Adipin-, die 6-Hydroxycapronsäure und/oder eines ihrer Gemische ist.

4. Verfahren nach einem der Ansprüche 1 bis 3, wobei die Temperatur während der Hitzebehandlung bei 130 - 300 °C liegt.

5. Verfahren nach einem der Ansprüche 1 bis 4, wobei die Lösung (a) bzw. (b) über einen Zeitraum von 10 Minuten bis 20 Stunden erhitzt wird.

6. Verfahren nach einem der Ansprüche 1 bis 5, wobei die Lösung in Gegenwart eines sauren Katalysators erhitzt wird.

7. Verfahren nach einem der Ansprüche 1 bis 6, wobei die Lösung (b) im Anschluß an die Hitzebehandlung einer üblichen Hydrierung unterworfen wird, so daß die Vorläuferverbindungen zu Hexandiol-1,6 umgesetzt werden.

8. Verfahren nach einem der Ansprüche 1 bis 7, wobei die abzutrennende Verunreinigung Cyclohexandiole sind.

9. Verfahren nach einem der Ansprüche 1 bis 8, wobei das Gewichtsverhältnis zwischen der (den) Carbonsäure(n) und den zu entfernenden Cyclohexandiolen größer gleich 1 : 1 ist.

10. Verfahren nach einem der Ansprüche 1 bis 9, wobei das Gewichtsverhältnis zwischen Wasser und den zu entfernenden Cyclohexandiolen größer gleich 5 : 1 ist.

## Claims

1. A process for separating impurities from aqueous solutions of 1,6-hexanediol or 1,6-hexanediol precursors, such as adipic and 6-hydroxycaproic acid, which comprises adding at least one carboxylic acid to a solution (a) of 1,6-hexanediol and subjecting this solution (a) or a solution (b) containing carboxylic acid(s) as precursor(s) of 1,6-hexanediol to a heat treatment at temperatures above room temperature in the absence of hydrogen.

2. A process as claimed in claim 1, wherein the added carboxylic acid(s) is or are one or more C₂-C₂₀-monocarboxylic acid(s), C₂-C₁₈-dicarboxylic acid(s) or a mixture thereof, and these mono- and dicarboxylic acids may contain aromatic radicals, cycloaliphatic radicals, heterocyclic radicals or halogen atoms.

3. A process as claimed in claim 1 or 2, wherein the added carboxylic acid is adipic acid, 6-hydroxycaproic acid or a mixture thereof.

4. A process as claimed in any of claims 1 to 3, wherein the temperature during the heat treatment is 130-300°C.

5. A process as claimed in any of claims 1 to 4, wherein solution (a) or (b) is heated over a period of from 10 minutes to 20 hours.

6. A process as claimed in any of claims 1 to 5, wherein the solution is heated in the presence of an acidic catalyst.

7. A process as claimed in any of claims 1 to 6, wherein, after the heat treatment, solution (b) is subjected to a conventional hydrogenation so that the precursors are converted into 1,6-hexanediol.

8. A process as claimed in any of claims 1 to 7, wherein the impurities to be separated off are cyclohexanediols.

9. A process as claimed in any of claims 1 to 8, wherein the weight ratio of the carboxylic acid(s) to the cyclohexanediols to be removed is greater than or equal to 1 : 1.

10. A process as claimed in any of claims 1 to 9, wherein the weight ratio of water to the cyclohexanediols to be removed is greater than or equal to 5 : 1.

## Revendications

1. Procédé d'isolement d'impuretés à partir de solutions aqueuses d'hexanediol-1,6 ou de composés précurseurs d'hexanediol-1,6, tels que de l'acide adipique et de l'acide 6-hydroxycaproïque, dans lequel le procédé comprend les étapes consistant à fournir à une solution (a) d'hexanediol-1,6 au moins un acide carboxylique et à soumettre cette solution (a) ou respectivement une solution (b) contenant comme composé(s) précurseur(s) de l'hexanediol-1,6 du ou des acides carboxyliques, à un traitement thermique à des températures supérieures à la température ambiante, en l'absence d'hydrogène.

2. Procédé suivant la revendication 1, dans lequel le ou les acides carboxyliques ajoutés sont un ou plusieurs acides monocarboxyliques en C₂ à C₂₀, acides dicarboxyliques en C₂ à C₁₈ et/ou un mélange de ceux-ci, ces acides monocarboxyliques et dicarboxyliques pouvant contenir des radicaux aromatiques, des radicaux cycloaliphatiques, des radicaux hétérocycliques et/ou des atomes d'halogène.

3. Procédé suivant l'une des revendications 1 et 2, dans lequel l'acide carboxylique ajouté est l'acide adipique, l'acide 6-hydroxycaproïque et/ou un de leurs mélanges.

4. Procédé suivant l'une des revendications 1 à 3, dans lequel la température pendant le traitement thermique est de 130-300°C.

5. Procédé suivant l'une des revendications 1 à 4, dans lequel la solution (a) ou respectivement (b) est chauffée pendant un espace de temps de 10 minutes à 20 heures.

6. Procédé suivant l'une des revendications 1 à 5, dans lequel la solution est chauffée en présence d'un catalyseur acide.

7. Procédé suivant l'une des revendications 1 à 6, dans lequel la solution (b) est, à la suite du traitement thermique, soumise à une hydrogénation courante de façon que les composés précurseurs soient transformés en hexanediol-1,6.

8. Procédé suivant l'une des revendications 1 à 7, dans lequel les impuretés à isoler sont des cyclohexanediols.

9. Procédé suivant l'une des revendications 1 à 8, dans lequel le rapport en poids entre le ou les acides carboxyliques et les cyclohexanediols à éliminer est supérieur à 1/1.

10. Procédé suivant l'une des revendications 1 à 9, dans lequel le rapport pondéral entre l'eau et les cyclohexanediols à éliminer est supérieur à 5/1.
